Europäisches Patentamt

European Patent Office

Office européen des brevets

(11) Publication number: **0 143 393**
**A2**

# EUROPEAN PATENT APPLICATION

(21) Application number: **84113715.1**

(51) Int. Cl.⁴: **A 61 K 31/725**

(22) Date of filing: **14.11.84**

(30) Priority: **25.11.83 US 555310**

(43) Date of publication of application: **05.06.85**
**Bulletin 85/23**

(84) Designated Contracting States: **AT BE CH DE FR GB IT LI NL SE**

(71) Applicant: MILES LABORATORIES, INC., 1127 Myrtle Street, Elkhart Indiana 46514 (US)

(72) Inventor: **Brown, Karen K., 10000 N.E. Harrison Drive, Kansas City Missouri 64155 (US)**
Inventor: **Cooper, Harold, Route 2 Box 181 B, Platte City Missouri 64079 (US)**

(74) Representative: **Dänner, Klaus, Dr. et al, c/o Bayer AG Konzernverwaltung RP Patentabteilung, D-5090 Leverkusen 1 Bayerwerk (DE)**

(54) The use of ultrapure hyaluronic acid to improve animal joint function.

(57) Hyaluronic acid having controlled molecular weight and substantially free of protein and nucleic acids can be prepared from a hyaluronidase-negative or hyaluronidase inhibited microbiological source and product is especially useful as a joint replacement fluid in mammals.

EP 0 143 393 A2

The present invention  is concerned generally with the
preparation, purification and use of hyaluronic acid and
its salts and specifically with the preparation of hyaluronic
acid from a microbiological source and its use in the
treatment of joint disorders in animals.

Hyaluronic acid is a naturally occurring high
molecular weight polysaccharide having an emperical formula
of $(C_{14} H_{20} N Na O_{11})n$ where $n > 1000$. The general
structure of hyaluronic acid is illustrated in Merck Index,
Ninth Ed. (3rd printing, 1978), at page 624.  It is well
known that hyaluronic acid and its salts, hereafter collectively referred to as HA, can be obtained from at least
three sources:  human umbilical cords, rooster combs and
certain bacterial cultures such as group A and C hemolytic
streptococci.  To the best of our knowledge, however, only
umbilical cords and rooster combs are used as sources for
commercially available HA.  This is somewhat surprising in
view of certain disadvantages associated with using those
two sources (e.g. relatively low yields, and labor
intensive processing and purification steps).

Since HA is found in aqueous and vitreous humor and the
synovial fluid of mammalian joints, there has been
considerable interest in obtaining purified HA for use as a
fluid replacement to correct pathological conditions in the
eye and in joints.  The preparation of HA from rooster

CUT 87

combs and human umbilical cords and its use in eye and
joint applications is described in U.S. Patent 4,141,973 to
E. A. Balazs.  That patent also provides a detailed review
of the technical literature describing the isolation,
characterization and uses of HA.

U.S. Patent 4,303,676, also to E. A. Balazs, describes
cosmetic formulations containing sodium hyaluronate
fractions in various molecular weight ranges made from
rooster combs.  U.S. Patent 4,328,803 to L. G. Pape
discloses the use of an ultrapure hyaluronic acid salt in
eye surgery.  The HA product used was a sodium hyaluronate
salt available under the registered trademark HYARTIL® from
Pharmacia, Inc. and obtained in commercial quantities from
rooster combs.

The only literature found which describes extraction of
hyaluronic acid from bacteria (see Kjem and Lebech, Acta
path. microbiol. scand. Sect. B, $\underline{84}$:162 - 164, 1976) uses a
media and process which are unacceptable for some purposes.
The described media will not support growth of most
Streptococci.  The described process begins with heat
killing the Streptococci.  This extracts the organisms,
releasing numerous internal contaminants which are more
likely to be reactive and which are difficult to remove
from the final product.  Therefore, it is likely the
resulting HA could not be used for injection into mammals.

Because the medical applications of HA require that the HA
be injected into a mammalian body (e.g. as a fluid replacement), it is very important that the injected products be
as pure as possible to avoid reactivity problems.  This
importance of purity is described in U.S. Patent 4,141,973
which describes an ultrapure HA product prepared from
rooster combs or, alternatively, from human umbilical
cords.  In addition to purity, it appears that control of
molecular weight of an HA product is very important (e.g.

CUT 87

the 4,141,973 patent suggests an average molecular weight of at least 750,000 and U.S. Patent 4,303,676 suggests having two distinct fractions of controlled molecular weight, one low and one high). Although there is a description of a high molecular weight (1,200,000) HA preparation of very high purity (i.e. less than 0.05 % protein) in a paper by Swann, Arch. Opthal. 88, pp. 544 - 8 (1972), we are unaware of any description of an HA product having the following advantages: (1) derivable from a microbiological source at relatively low costs, in high yields, and with low reactivity upon injection; (2) having a desirably high and closely controlled average molecular weight; and (3) being substantially free of protein and nucleic acid impurities. Quite surprisingly, we have found it is now possible to prepare such a product. Details of its preparation, characterization, and use are described below.

The method of improving joint function in an animal comprises replacing at least a portion of the animal's existing joint fluid with the sterile, purified hyaluronic acid preparation described herein.

The method of preparing the ultrapure HA preparation of controlled high average molecular weight comprises the steps of culturing an HA-producing, hyaluronidase-negative or hyaluronidase-inhibited organism under conditions sufficient to enhance HA content in the culture, releasing the HA from the cells, and purifying the released HA to remove substantially all protein and nucleic acids. In preferred embodiments, the HA is prepared from a group C streptococcal organism (such as Strep. equi), preferably cultured in a medium free of extraneous proteins, with the final product being a sterile preparation having a tightly controlled average molecular weight and containing less than about 1.25 mg/ml of protein (preferably less than about 0.10 mg/ml) and less than about 45 µg/ml of nucleic acids (preferably less than about 5 µg/ml), and is used (for example) as a synovial fluid replacement in mammals.

As shown in the examples below, the HA product of this disclosure is different from commercially available HA products in that it is made from a hyaluronidase-negative or hyaluronidase-inhibited microbiological source, has a tightly controlled average molecular weight and, very importantly, is substantially free of proteins and nucleic acids, and contains no chondroitin sulfate, all of which are considered undesirable contaminants in any product intended for replacement of an animal fluid. As used herein, the expression "substantially free of", when applied to the

CUT 87

protein and nucleic acid content of an HA preparation,
means that the protein content of the product is less than
about 1.25 mg/ml (preferably less than about 0.10 mg/ml)
and the nucleic acid content is less than about 45 µg/ml
(preferably less than about 5 µg/ml). The expression
closely controlled high molecular weight means that at
least 98% of the HA is within a given high average molecular weight range (preferably from about 2.0 million (MM) to
about 4.0 MM, and represented by an essentially single,
substantially symetrical molecular weight distribution peak
via the HPLC technique described below). Hyaluronidase-
negative means that no measurable amounts of extracellular
hyaluronidase (able to degrade HA to small molecules) are
associated with the organism. Hyaluronidase-inhibited
means that an inhibitor such as heat or enzyme inhibitors
has been used so as to eliminate the breakdown of HA to
smaller molecules.

In the examples below, the purity and efficacy of the HA
prepared according to this disclosure was demonstrated and
compared with existing commercial HA products by using the
HA as a joint fluid replacement in the horse. It can be
appreciated that the product can also be used in any
applications in other mammals, including humans, which call
for the use of a highly purified HA preparation as a fluid
replacement or for other purposes.

In the specific illustrative preparation steps given below,
we used a known group C streptococcal HA producer (Strep.
equi) and, by novel culturing and purification techniques,
we demonstrate how it is now possible to obtain an
ultrapure product of controlled molecular weight with very
large yields (e.g. Avg. 31% w/w vs 0.079% w/w from rooster
combs), thus providing the advantages of higher purity and
better product molecular weight control with the clear
advantages of economy. A sample of the Strep. equi strain
used below has been deposited with the American Type

CUT 87

- 6 -                    0143393

Culture Collection, Rockville, MD 20852 as A.T.C.C. No.
39,506.

As noted above, it has long been known that HA is a major
component of the capsule of Types A and C streptococci.  A
representation of a streptococcal organism illustrating the
capsular location (hyaluronate capsule) of HA has been
published by Beachey and Stollerman and is shown in Trans.
Assoc. Am. Physicians Phila., 85:212 - 221, 1972.

As has been demonstrated, the HA capsule is the outermost
component making up a large portion of the total streptococcal cell.  During growth of a streptococcal culture such
capsule may be observed after india ink staining of the
culture as a bright halo around each cell.  As a result of
this procedure it has been determined that maximum capsule
production is obtained from several strains of streptococci
grown in controlled fermentation systems by about 4 to 6
hours after the beginning of log phase growth.  During late
log and stationary phase the visible capsule disappears.
It is known that in some strains of streptococci this
disappearance of capsule is due to enzyme degradation by
hyaluronidase.  In long-term fermentation studies (2 - 5
days growth) with at least one group C streptococci (Strep.
equi) in which pH, temperature, and glucose levels were
controlled, we have determined that yields of HA can be
substantially increased even though, surprisingly, the
capsule is not visibly apparent in the culture.  In such
studies it was concluded that this strain probably lacks
hyaluronidase.  Thus, when extracellular hyaluronidase
negative strains of streptococci are grown under controlled
conditions specific for HA production, yields of an
extraordinarily pure, high specific molecular weight HA
have been reached and this lack of the hyaluronidase enzyme
is considered an important aspect of the HA preparation.

CUT 87

As indicated, this invention describes the process for
obtaining such high yields of extraordinary quality, high
molecular weight hyaluronic acid from bacteria such as
Streptococci and a method of use of such HA to replace
synovial fluid from diseased joints in order to reduce
lameness and swelling of such joints.

In our best examples, fermentation of a Group C streptococcus was continued at a pH between 7.0 and 7.2 for from
24 hours to 120 hours at 37° C. A special chemically
defined media, described by I. van de Rijn in _Infect. and
Immun._, _27_:444 - 448, 1980, was used for growth. This
media is preferable since it contains no extraneous
proteins which would have to be removed in later purification steps. Dextrose is added at 24 hour intervals to
serve as a carbon source. The culture may be grown under
intermittent pH control, adjusting to pH 7.6 at each
addition of dextrose. Approximately 12 hours before
harvest, the pH controller is shut off and the pH is
allowed to drop to 6.5 to 6.8 where the culture stops
growing. This allows more efficient centrifugation and
somewhat better yields of hyaluronic acid.

At harvest, at least 0.01% sodium lauryl sulfate (SLS), or
an equivalent amionic detergent, is added to the culture in
order to release hyaluronic acid from the cells. After at
least 15 mins., the SLS culture is titrated for floc
formation after addition of varying amounts of a 10%
solution of hexadecyltrimethylammonium bromide, or an
equivalent non-ionic detergent.

Generally, between 100 ml and 400 ml of this second
detergent is added to 10 l of SLS culture in order to
precipitate HA and SLS. After allowing at least 1 hour for
maximum floc formation the precipitate is collected via
centrifugation or sieve filtration. This precipitate is
then solubilized in 2M $CaCl_2$ of approximately 1/10 to 1/20

CUT 87

the original volume. Solubilization is carried out for at least 6 hours at 4° - 30° C. The resulting suspension is centrifuged or sieve filtered in order to remove the precipitate which contains cellular contaminants and both detergents. The supernate is saved and extracted with 2 volumes of a suitable alcohol (95% EtOH or 99% isopropanol preferred). A gelatinous precipitate forms which is collected after at least 1 hour via centrifugation or sieve filtration. The precipitate is solubilized overnight at 4° C - 10° C in deionized, distilled water approximately 1/10 to 1/20 the original volume. The suspension is centrifuged or sieve filtered to remove the precipitate. One percent NaCl (w/v) is added to the supernate and dissolved. Then, 2 volumes of an appropriate alcohol are added to reprecipitate the HA. Such precipitate is allowed to settle at least one hour after which it is collected via centrifugation or sieve filtration.

The solubilization of the HA in water followed by 1.0% NaCl addition and alcohol precipitation are continued in increasingly smaller volume (1/20 - 1/100 original volume) until the HA-water solution is clear. This usually requires at least four additional alcohol precipitation steps. An outline of the process is shown below.

OUTLINE OF PROCESS FOR EXTRACTION
OF BACTERIAL HYALURONIC ACID

1.    Grow Streptococcus organism

2.    1 ml/l SLS 10%

3.    10 - 40 ml/l
      Hexadecyltrimethylammonium bromide 10%

4.    Collect ppt.

5.    Solubilize in 2M CaCl$_2$

6.    Collect supernate

CUT 87

7.  2 Vol. alcohol
    (ppt. HA, some nucleic acids, some protein)

8.  Collect ppt.

9.  Solubilize ppt. in DI-$H_2O$

10. Discard undissolved ppt.

11. Collect supernate

12. 1% NaCl
    2 Vol. alcohol
    (ppt. HA)

13. Collect ppt.

14. Solubilize in DI-$H_2O$

15. Discard ppt.
    Collect supernate

16. 1% NaCl
    2 Vol. alcohol
    (ppt. HA)

17. Collect ppt.

18. Solubilize in DI-$H_2O$

19. Discard ppt.
    Collect supernate

20. Filter - protein binding type (e.g., nitrocellulose)
    (remove some of the minimal protein remaining)

21. 1% NaCl
    2 Vol. alcohol
    (ppt. HA)

22. Collect ppt.

23. Solubilize 0.15M phosphate buffered saline pH 7.2

24. Adjust to 1% HA by spectrophotometric assay

25. Sterilize with 0.1% betapropiolactone
    4 - 10° C 24 - 48 hours

26. Hydrolize betapropiolactone
    37° C 24 - 48 hours

27. Fill Syringes

CUT 87

The final steps of product preparation may involve washing with 95% EtOH and 99% acetone followed by drying under vacuum. The dried HA is resuspended in 0.15M sodium phosphate buffer to a concentration of 1.0%. This may be filter sterilized through a final 0.45μ nitrocellulose type filter and/or sterilized in final bulk form with 0.1% betapropiolactone. The betapropiolactone sterilization is conducted at 4° C for 24 - 48 hours followed by hydro-lization of the betapropiolactone at 37° C for 24 - 48 hours. The final product contains 10 mg/ml HA in 0.15M sodium phosphate buffer. When these steps are followed, HA of the highest purity is obtained in high yield (> 99.90% HA).

As an example of yield, an average 10 1 fermenter of <u>Strep. equi</u> produces 5 g to 7 g dry weight of cells and 1.0 g to 2.5 g dry weight of HA. Yield is therefore between 14.3% and 50% (w/w). Yields of HA from extraction of rooster combs as in U.S. Patent 4,141,973 are reportedly around 0.079%.

It should be noted that a latter-stage filtration through a suitable protein-binding filter (for example a nitrocellulose filter) is necessary in order to remove reactivity of the final product HA. Other types of filters (plain cellulose and cellulose acetate) do not adequately remove reactivity as observed in the horse joint injection test. It is thought that this step removes the minute quantity of reactive proteinaceous material remaining in the HA.

The purity of our bacterial-derived HA has been proved via a colorimetric protein assay, U.V. spectrophotometry, HPLC, and slab gel electrophoresis. Initial experiments involved quantitation of protein contamination as measured via the BIO RAD Protein Assay. This method can detect levels of protein as low as 200 ug/ml. Table I lists the results of

0143393

testing aqueous 1.0% solutions of hyaluronic acid extracted from four different fermenters of <u>Strep. equi</u>.

<div align="center">

### TABLE I
BIO RAD Protein Assay Results

</div>

| Sample | O.D. at 595 mμ | Concentration of Protein |
|--------|----------------|--------------------------|
| Ferm 1 | 0.00,0.000 | < 200 ug/ml |
| Ferm 2 | 0.00,0.010 | < 200 ug/ml |
| Ferm 3 | 0.00,0.005 | < 200 ug/ml |
| Ferm 4 | 0.00,0.005 | < 200 ug/ml |

According to such data, the protein content of a 1.0% bacterial-derived HA solution may be as high as 0.001%.

A second method of determining protein, peptide, and/or amino acid content is UV absorption at 280 mμ. A known concentration of Bovine Serum Albumin was used as a control. Table II compares these results with UV absorption of the same solutions at 257 mμ. Absorption at 257 mμ represents contamination with nucleotides or nucleic acid such as DNA and RNA. It is noted that spectrophotometric absorption at 280 mμ detects more protein contamination than the Bio RAD assay. The 1.0% solutions of bacterial-derived HA contain at most 0.12% contaminants which absorb at 280 mμ. Since these same solutions contain no nucleic acid contamination, the purity is in the range of at least 99.88%. In this respect it is notable that amino acid analysis of similarly extracted HA indicated the presence of <0.04% protein. This would mean that the HA purity is as high as 99.96%. This is compared with the purity of commercially available rooster comb derived HA (HYARTIL® available from Pharmacia or Hyalovet™ available from Trans Bussan) which, according to our tests, have purities in the range of 99.78% to 99.86% respectively.

CUT 87

## TABLE II

PROTEIN AND NUCLEIC ACID CONTAMINATION OF 1.0%
HYALURONIC ACID AS MEASURED BY UV SPECTROPHOTOMETRY

| | UV Absorbence | | Concentrations | |
| | 0.0. at 280 mµ | 0.0. at 257 mµ | Protein mg/ml | Nucleic Acid ug/ml |
| --- | --- | --- | --- | --- |
| Sample | | | | |
| Ferm 1 | 0.45 | 0.00 | 0.66 | 0.0 |
| Ferm 2 | 0.83 | 0.00 | 1.22 | 0.0 |
| Ferm 3 | 0.75 | 0.00 | 1.10 | 0.0 |
| Ferm 4 | 0.47 | 0.00 | 0.69 | 0.0 |
| Miles Labs. (Rooster Comb) low purity | 1.16 | 1.31 | 1.70 | 48.5 |
| Pharmacia HYARTIL® | 0.91 | 1.63 | 1.33 | 60.3 |
| Trans Bussan Hyalovet™ | 1.27 | >2.0 1/30 dil.=0.27 | 1.86 | >74<300 |

Further studies on purity were conducted with the
bacterial-derived HA.  Effectiveness of two alcohol purification processes were followed spectrophotometrically at
280 mµ, 257 mµ, and 195 mµ.  Absorbence at 195 mµ
represents the actual absorbence of HA and is lineraly
related to concentration of HA.  Table III shows optical
density results whereas Table IV converts all readings to
concentrations of protein, nucleic acid and HA.  These two
more recent fermenters yielded bacterial-derived HA which
was ·99.99% pure using either 95% E+OH for extraction or
using 99% isopropyl alcohol.

## TABLE III
### PROOF OF PURIFICATION OF HYALURONIC
### ACID SOLUTIONS – UV ABSORBENCE

| Purification Step | U.V. Absorbence | | | | | |
| | O.D. at 280 mµ | | O.D. at 257 mµ | | O.D. at 195 mµ | |
| | ETOH | Isopropyl | ETOH | Isopropyl | ETOH | Isopropyl |
|---|---|---|---|---|---|---|
| 1st $H_2O$ Solution | 0.480 | 0.442 | 0.456 | 0.341 | 0.740 | 0.643 |
| 2nd $H_2O$ Solution | 0.215 | 0.288 | 0.204 | 0.230 | 1.00 | 0.898 |
| 3rd $H_2O$ Solution | 0.215 | 0.155 | 0.191 | 0.182 | 1.76 | 1.82 |
| 4th $H_2O$ Solution (Final 1%) | 0.078 | 0.070 | 0.140 | 0.120 | 1.90 | 1.82 |

## TABLE IV
### PROOF OF PURIFICATION – PROTEIN AND NUCLEIC ACID CONCENTRATIONS COMPARED WITH HYALURONIC ACID CONCENTRATION

| Purification Step | Concentration | | | | | |
| --- | --- | --- | --- | --- | --- | --- |
| | Protein mg/ml | | Nucleic Acid µg/ml | | Hyaluronic Acid mg/ml | |
| | ETOH | Isopropyl | ETOH | Isopropyl | ETOH | Isopropyl |
| 1st $H_2O$ Solution | 0.52 | 0.66 | 17.3 | 12.8 | 4.0 | 3.5 |
| 2nd $H_2O$ Solution | 0.32 | 0.44 | 7.6 | 8.5 | 5.4 | 4.9 |
| 3rd $H_2O$ Solution | 0.32 | 0.22 | 7.0 | 6.9 | 9.3 | 9.8 |
| 4th $H_2O$ Solution (Final 1% HA in $H_2O$) | 0.08 | 0.10 | 5.0 | 4.3 | 10.2 | 9.8 |

Slab gel electrophoresis was used to further analyze the various 1.0 % hyaluronic acid preparations listed in Table II for nucleic acids. Such a technique can differentiate DNA from RNA. A 0.8% low endosmosis agarose containing 2 ug/ml ethidium bromide was used in conjunction with short wave UV light in order to visualize the nucleic acids after electrophoresis. DNA being of a much larger molecular weight remains near the origin whereas RNA migrates with the buffer front. Twenty-five ul samples were electrophoresed 18 hours at 90 volts in a Canalco Slab Gel apparatus. Results indicated no detectable nucleic acids in any of our four preparations or the HYARTIL® product. The Hyalovet™ product showed a significant amount of nucleic acid in the form of RNA.

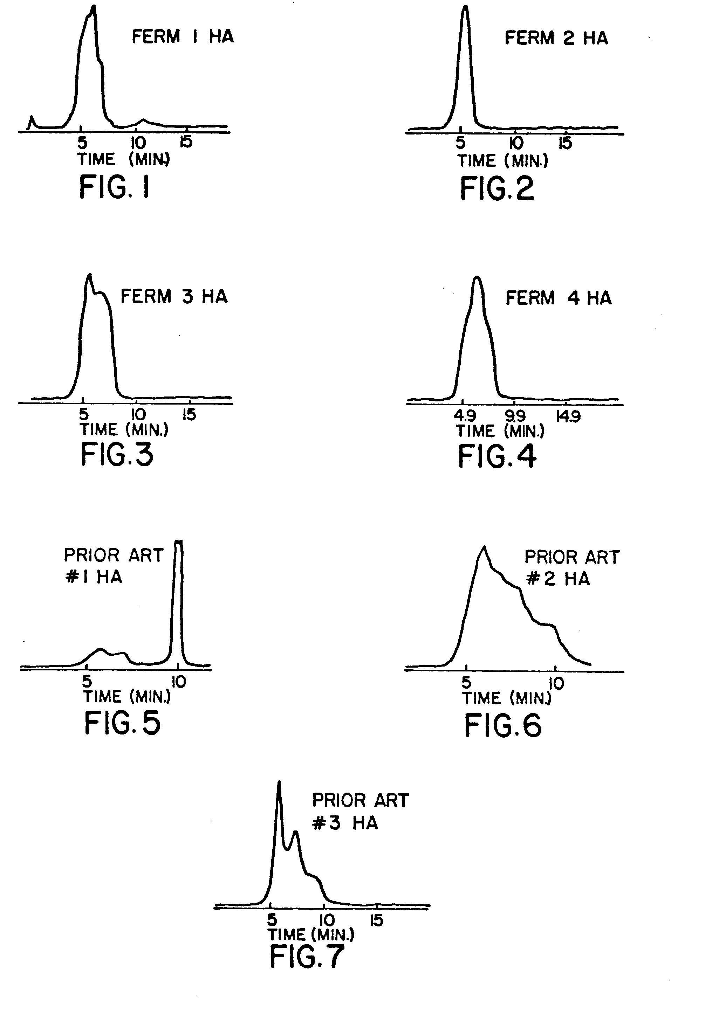

High Performance Liquid Chromatography (HPLC) was used to analyze the molecular weight of the various HA preparations. This is a newer and more accurate method than viscometry as mentioned in the patent no. 4,141,973. To date, only one column (Waters Micro Bondagel/E-High Å) has been used for molecular weight determination. It has been impossible to run aqueous standards in the molecular weight range required along with test samples in order to determine accurate molecular weights. However, we have determined relative molecular weights based upon retention times on the column. Theoretically, with this procedure, the earlier the time of peak detection the higher the molecular weight. The column has a retention time of 10 min. with minimum and maximum molecular weight capabilities between 15,000 and 7,000,000. Figures 1 - 4 show the HPLC tracings for our first four fermenter preparations. Miles Hyaluron, Pharmacia HYARTIL®, and Trans Bussan Hyalovet are shown in Figures 5 - 7. The retention times of the peaks and shoulders have been determined and relative molecular weights have been calculated based on a linear relationship between molecular weight (15,000 to 7,000,000) and

CUT 87

retention time (0 - 10 minutes). Such relative molecular weights are listed in Table V. As can be seen in Figures 1 - 4, the HPLC separated HA of this disclosure results in an essentially single, substantially symmetrical high molecular weight (avg. higher than about 2 MM) distribution peak. In Figures 1 - 4 at least about 98% of the HA content is within the single peaks shown. Such close control of high molecular weight distribution is not shown in existing commercial products as illustrated in Figures 5 - 7. Figure 5 (Prior Art #1) illustrates the HPLC tracing for the Miles Hyaluron HA product. Figure 6 (Prior Art #2) represents the Hyalovet product and Figure 7 (Prior Art #3) represents the HYARTIL® product. It is thought that this close control of final molecular weight range may be due to the simplicity of the extraction procedure requiring minimal shear-producing steps as well as to the lack of hyaluronidase which could degrade high molecular weight HA.

## TABLE V
### Relative Molecular Weights of Hyaluronic
### Acid Moieties in Various Preparations

| Sample | Relative Molecular Weights in Millions | |
|---|---|---|
| | Range | Average |
| Fermenter 1 | 2.2 - 3.9 | 2.8 |
| Fermenter 2 | 2.5 - 4.0 | 3.8 |
| Fermenter 3 | 1.7 - 2.8 | 2.4 |
| Fermenter 4 | 1.1 - 3.9 | 2.6 |
| Hyaluron | 0.015 - 3.0 | 0.015 |
| HYALOVET | <0.010 - 3.7 | .015, 1.8, 3.7 |
| HYARTIL® | <0.010 - 3.8 | 1.9 |

As noted above the relative molecular weight range of HA moieties found in bacterial-derived HA is narrow with the

CUT 87

majority (98%) measuring between about 1,100,000 or 2,200,000 and 4,000,000. Via the same method, Hyalovet contains three distinct molecular weight moieties of 2,700,000; 1,700,000 and 300,000. Finally, the HYARTIL® product contained an array of HA molecular weights from <10,000 to 3,700,000. As shown, the HYARTIL® product contains the widest variation of molecular weight sizes.

From the various analytical tests described herein, it has been determined that HA extracted from bacteria via a simple method is purer than three commercial products made from either rooster combs or umbilical cords. The latter are produced via a complex process which is inefficient yielding only 0.079% HA. This is compared with HA extracted from streptococci which can reach yields as high as 50% w/w.

## Joint Fluid Replacement

Hyaluronic acid prepared from bacteria as described herein has been tested for reactivity in tibiotarsal and radial-carpal joints of horses. The following clinical index test was devised in order to measure reactivity of HA preparations post intra-articular injection of horses.

The test protocol is as follows:

1.  Assess normal movement of joint to be injected. Assign lameness indices from 0 to 5 according to the following definitions.

Lameness Index

0 = No lameness

1 = Slight lameness - moderate

2 = Noticeable lameness - moderate

3 = Obvious lameness

4 = Severe lameness - reluctant to move or bear weight

5 = Cannot bear weight.  If down, animal is unable to rise.

2.   Sedate horse (e.g. with Rompun® sedative).

3.   Shave hair around the joint area to be injected.

4.   Determine Swelling Observation Index according to the following definitions.

Swelling Observation Index

0 = No swelling

1 = Nothing obvious - palpable fluid

2 = Slightly noticeable - palpable fluid

3 = Noticeable swelling of entire joint

4 = Severe swelling at injection site

5 = Severe swelling involving more than the joint alone.

5.   With cloth tape measure, measure joint circumference immediately anterior to the anterior aspect of the third metatarsal (tibiotarsal joint) or immediately distal to the protuberance of the accessory carpal bone that is around the radial carpal bone (carpal joint).

The exact circumference of the joint (in millimeters) before and after injection is recorded.  A difference between the circumference each day post injection and

CUT 87

the original circumference is calculated. If the difference is greater than 1.0 cm, the **exact** measurement is added to the other two **index** values in order to determine the clinical index.

6.  Remove joint fluid (1.0 - 2.0 cc) prior to injection with a 3.0 cc syringe with a 20 - 22 ga. X 1" needle.

7.  Inject joint with 2.0 cc of a 1% preparation of hyaluronic acid being evaluated for reactivity. For this injection, use a 3.0 cc syringe with the same needle (exchanging syringes only) as used to remove joint fluid. This is done so as to reduce trauma to the joint as much as possible.

8.  Apply digital pressure to the injection site for 1 to 3 minutes after injection. This is done to prevent backflow of the very viscous HA.

9.  Observations and measurements are made for four consecutive days post injection, then on day 7.

10. The Clinical Index (CI) is calculated as follows:

    Total Lameness Index (TLI) = Sum of Daily Lameness Indices

    Total Swelling Index (TSI) = Sum of Daily Swelling Observations + Sum of Joint Circumference Measurements Greater Than 1.0 cm.

    CI = TLI + TSI

11. Interpretation

    Joint injection alone causes trauma with development of some swelling and lameness. This was proven by

CUT 87

evaluating numerous joints injected with phosphate buffered saline (PBS) and some joints in which only fluid was removed.  CIs were calculated on these traumatized joints.  They varied from 0 to 18.7 among 56 joints.  However, the average CIs in the three separate studies of traumatized joints showing these wide individual variations were 0.7, 5.3, and 3.4.  It is thus suggested that an average CI value of 6.0 or less in at least 10 joints could be expected from injection trauma.  On this basis, we have assigned a 10 - joint average CI value of 6.0 or less as acceptable in the horse joint reactivity test for evaluation of HA preparations.  Any product showing a 10 - joint average of CI of >6.0 is unacceptable. Using these criteria, several HA preparations were tested.  Results are shown in Table VI.

## TABLE VI

Evaluation of Hyaluronic Acid Preparations
by the Horse Joint Reactivity Test

| Preparation | No. of Joints | Average CI | Acceptability of Preparation |
|---|---|---|---|
| Microbiological Source HA Filtered | 14 | 4.6 | Acceptable |
| through Nitrocellulose | 13 | 5.5 | Acceptable |
| Microbiological Source HA Non-filtered | 11 | 16.4 | Unacceptable |
|  | 6 | 17.4 | Unacceptable |
| Prior Art #1 Purified and Filtered | 10 | 6.2 | Unacceptable |

The microbiological source HA listed in Table V was that obtained from fermenters 1 - 4 as described previously.  It is noteworthy that this material is acceptable for joint injection after nitrocellulose filtration but not prior to such filtration.  On the other hand, Prior Art #1 (see

CUT 87

Figure 5) is on the borderline of being unacceptable even after nitrocellulose filtration. Evidently, the reactive proteinaceous load in the Prior Art #1 preparation is too great to be removed via the protein binding filtration step.

The same Clinical Index can be used to evaluate efficacy of treatment of diseased joints with HA. In this test system clinical symptoms are induced in joints with intra-articular injection of complete or incomplete Freund's adjuvant. This adjuvant produces first an acute and then a chronic pathology of the joint characterized by extreme lameness and swelling which does not appear to reverse itself within two months.

Some such efficacy studies have been conducted on the bacterial-derived HA Experiments.

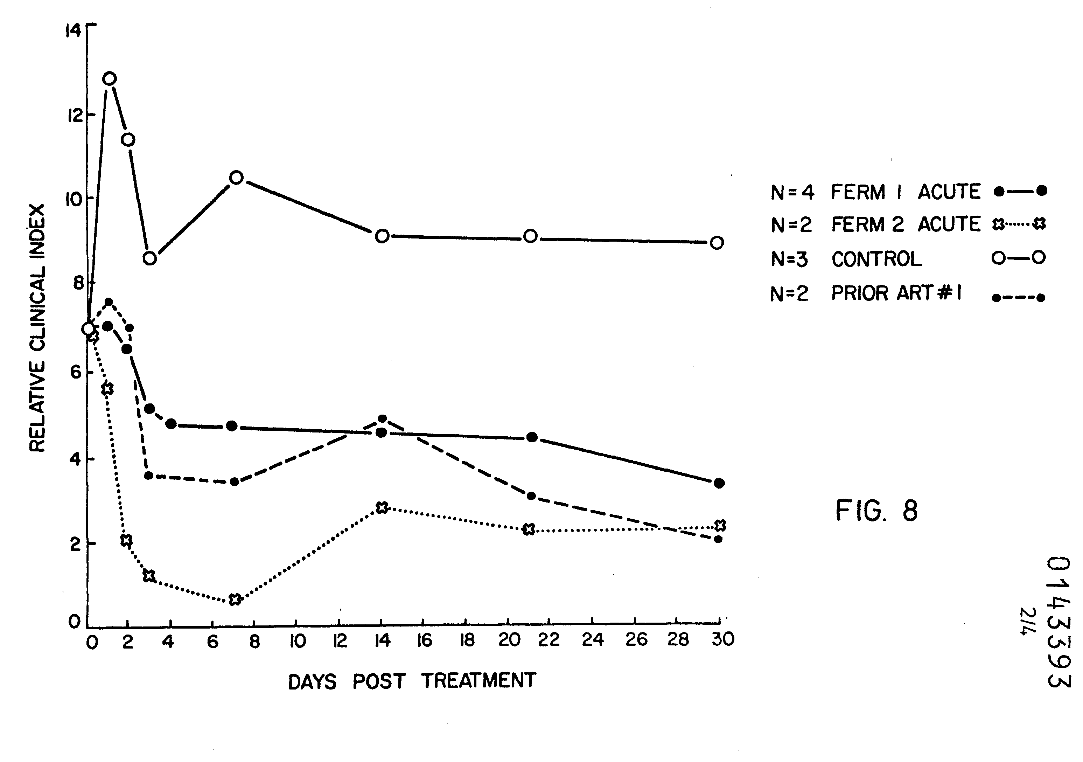

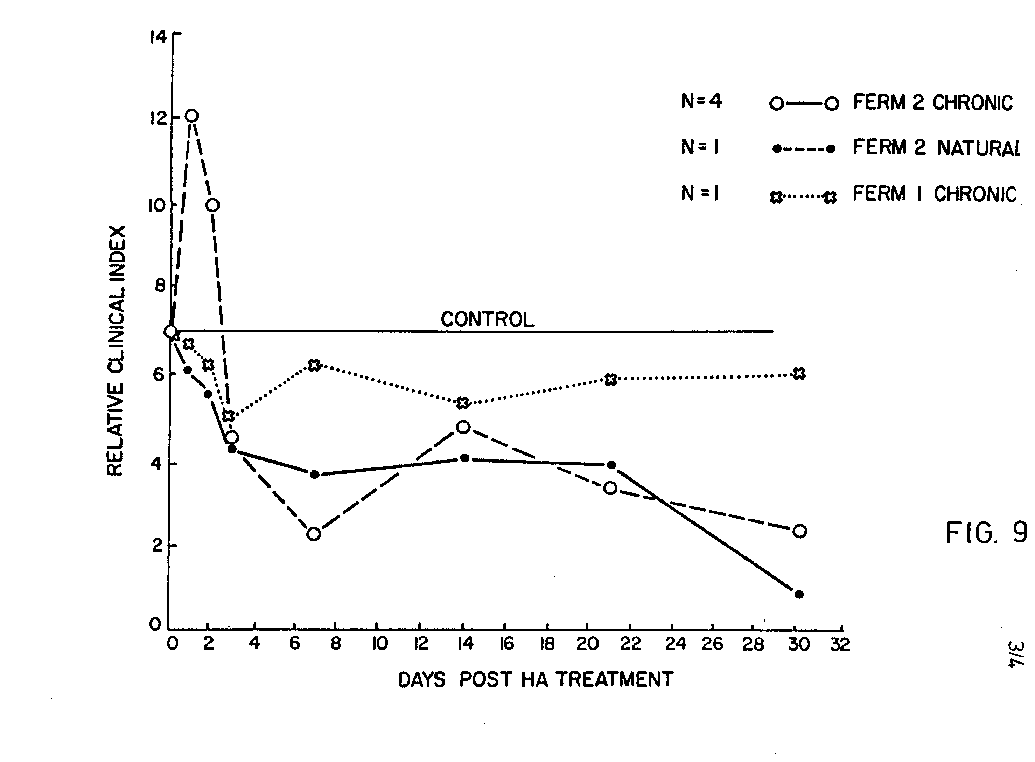

Experiments were designed to evaluate the effect of removing some of the joint fluid from adjuvant induced pathological joints and replacing it with bacterial-derived HA. This was done with both acute joints (HA injection within three days of Freund's injection) and with chronic joints (HA injected within 12 to 34 days of Freund's injection). Clinical Index evaluation was begun the day of adjuvant injection and continued for four days following the HA injection after which weekly observations were made for three weeks. Figures 8 and 9 display the results over 30 day periods.

Figure 8 represents the acute situation. The zero day readings were all adjusted to seven on the relative index scale so that comparisons could be better visualized. Zero day represents three days post Freund's injection in the acute joints. Figure 8 then portrays the change in Clinical Index for the first 30 days post injection with HA from fermenters 1 and 2 and Prior Art #1 after further

CUT 87

purification. These results are compared with similar
adjuvant injected joints left untreated (control). It is
notable that the control horses continually worsen through
day post Freund's injection before showing some improvement
on their own. However, this improvement does not reach the
starting level by day three and by day four appears to be
plateauing. This is the typical picture for induction of
chronic pathology. A significant improvement in acute
symptoms is observed after injection of HA.

The chronic situation is represented by Figure 9. Horses
which had been injected with Freund's complete or incomplete adjuvant 12 - 34 days prior to HA treatment can serve
as their own controls since these horses had been stable
for at least seven days prior to day zero. The control
line represents these control index levels. Again,
immediate clinical improvement is noted after treatment
with HA. Longer term observation of these horses has
indicated that the improvement tends to plateau. Therefore, it is expected that more than one treatment may be
necessary.

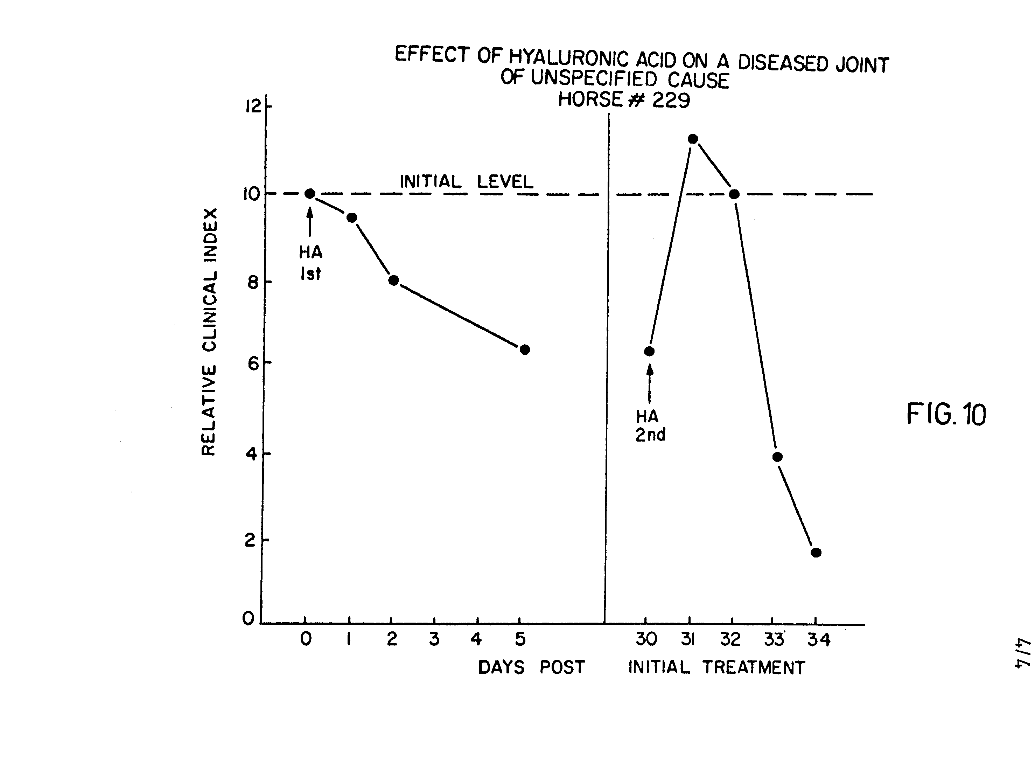

One horse, entered our study with a diseased joint of
unspecified cause. As indicated in Figure 10, two
injections of bacterial-derived HA were administered to
this joint 30 days apart. In this case, the zero day
readings were adjusted to 10 in order to display the
complete treatment response. Immediate improvement was
noted after both the first and second, injections of HA.
The improvement after the first injection was followed by
some return of pathology as indicated by swelling only.
After the second HA injection joint swelling was eliminated
and has not returned within three months post treatment.

From the data presented herein it is obvious that ultra
pure bacterial-derived HA is nonreactive in horse joints
and displays efficacy for reversing lameness and/or swelling in diseased joints.  Since bacterial-derived HA as described herein is purer than any commercially available product, including those used in opthalmalogical treatments, it is highly probably that these HA preparations could also be used to replace vitreous humor of the eye during surgery.  They should be able to substitute for any other use applied to the rooster comb or umbilical cord HA.

Given the above disclosures, it is thought that numerous variations in our HA production methods and uses will occur to those skilled in the art.  Thus, it is intended that the above-described examples should be construed as illustrative only and that the scope of the invention disclosed herein should be limited only by the following claims.

CUT 87

- 24 -                    0143393

<u>WE CLAIM:</u>

1.    A method of improving joint function in an animal comprising replacing at least a portion of the animal's existing joint fluid with a sterile, purified hyaluronic acid preparation being substantially free of proteins and nucleic acids and having a closely controlled high molecular weight distribution as determined by HPLC.

2.    The method of claim 1 wherein the protein content is less than about 1.25 mg/ml, the nucleic acid content is less than about 45 µg/ml and the preparation, when subjected to HPLC, is characterized by an essentially single, substantially symmetrical distribution peak.

3.    The method of claim 2 wherein the protein content is less than about 0.10 mg/ml and the nucleic acid content is less than about 5 µg/ml.

4.    The method of claim 3 wherein the hyaluronic acid preparation is bacterial-derived.

5.    The method of claim 4 wherein the hyaluronic acid is derived from a group A or group C hemolytic streptococcal organism.

6.    The method of claim 5 wherein the hyaluronic acid is derived from a <u>Streptococcus</u> <u>equi</u> organism.

7.    The method of claim 5 wherein the animal is a horse and the joint fluid replacement is in an extremity joint.

8.    The method of claim 7 wherein the hyaluronic acid has a Clinical Index of less than 6.0 as determined by the Horse Joint Reactivity Test described herein.

CUT 87

FERM 1 HA — FIG. 1

FERM 2 HA — FIG. 2

FERM 3 HA — FIG. 3

FERM 4 HA — FIG. 4

PRIOR ART #1 HA — FIG. 5

PRIOR ART #2 HA — FIG. 6

PRIOR ART #3 HA — FIG. 7

FIG. 8

N=4   FERM I  ACUTE ●——●
N=2   FERM 2  ACUTE ✕·······✕
N=3   CONTROL ○——○
N=2   PRIOR ART #I ●----●

0143393
2/4

FIG. 9

N=4    O——O   FERM 2 CHRONIC
N=1    ●----●   FERM 2 NATURAL
N=1    ✳······✳   FERM I CHRONIC

CONTROL

RELATIVE CLINICAL INDEX

DAYS POST HA TREATMENT

EFFECT OF HYALURONIC ACID ON A DISEASED JOINT
OF UNSPECIFIED CAUSE
HORSE # 229

FIG. 10